# EUROPEAN PATENT SPECIFICATION

(11) **EP 3 586 726 B1**
(45) Date of publication and mention of the grant of the patent: **11.05.2022**
(21) Application number: 18305795.9
(22) Date of filing: 22.06.2018
(51) Int. Cl.: A61B 5/00

(54) **DEVICE FOR IMAGING BLOOD VESSELS**
VORRICHTUNG ZUR BILDGEBUNG VON BLUTGEFÄSSEN
DISPOSITIF D'IMAGERIE DE VAISSEAUX SANGUINS

(43) Date of publication of application: 01.01.2020
(73) Proprietor: Université Paris Cité, 75006 Paris (FR); Centre National de la Recherche Scientifique - CNRS, 75016 Paris (FR)
(72) Inventor: FLEURY, Vincent, 75013 PARIS (FR)
(74) Representative: Regimbeau

(56) References cited:
- EP-A2- 1 494 579
- US-A1- 2005 180 636
- US-A1- 2007 217 663
- US-A1- 2017 100 037

## Description

### TECHNICAL FIELD

The present invention relates to a device for imaging blood vessels, more particularly capillaries of a living tissue such as the chicken Yolk-Sac, the Chorioallantoic membrane or the retina.

### BACKGROUND OF THE INVENTION

Vascular pathologies, or simply normal aging of the vascular architecture, are a major cause of death in western countries. Also, neoangiogenesis and vascularization is one possible therapeutic target of malignant tumors, which strive to develop by stimulating vascular development. Understanding vascular development is therefore of importance. It has been known for long that the formation of a functional vasculature is a dynamic phenomenon. Formation of a functional vasculature in model systems such as the chicken Yolk-Sac or the Chorioallantoic Membrane (CAM) generally takes a few days. Formation of a functional vasculature is a multiscale process, with rapid developmental changes (for example hours) from the capillary level up to the main vessels. Therefore, understanding the formation of the vessel trees requires a multi-scale approach, especially in terms of imaging. In particular, it is generally observed that arteries A and veins V avoid themselves and interdigitate. This dense interlace with narrow approach of arteries and veins is crucial for proper blood flow across capillaries and normal tissue physiology. Conversely, direct A-V connections are detrimental in that, in presence of direct shunts or fistulae, blood does not flow through capillaries. In such a case, molecular or gaseous (O₂, CO₂) exchanges hardly occur. The interdigitating structure is particularly striking in the CAM, which can be easily observed in vivo by shell-less experimentation. The origin of this vascular interlace is not understood.

Imaging living tissues generally involves the use of fluorescence dyes (as labels), fixation of the tissue, cast of the tissue and/or staining. These methods are not direct and can be expensive in the case of fluorescence. In the case of casting, the evolution of the same tissue cannot be observed along time. Finally, these methods cannot assess quantitatively the flow of cells, especially erythrocytes, in the vessels of the observed tissue.

EP 1494579 discloses a system for imaging surface layers of organs of interest, for example the retina. The system is configured for imaging moving bodies, such as cells, in a static background. Differential images of regions of changed intensity levels are calculated. The intensity level changes may, for example, come from the absorption of light by erythrocytes circulating in the vessels. The system is furthermore configured for superimposing the region of changed intensity so as to generate a map of vascular path positions in said region. The system can then image vessels without labelling nor fixing the observed tissue. The system is also configured to align the different images of a sequence before superimposing the region of changed intensity so as to compensate for a movement of a small amplitude coming from the camera or from the living tissue.

However, the system is not adapted for imaging living tissues in movement. The alignment of the images of a moving living tissue as disclosed by EP 1494579 can lack robustness in the case of a moving body. The image calculated by superimposition can be blurry or not representing the blood vessels. The alignment process can also fail if the background is moving.

### SUMMARY OF THE INVENTION

A device for automatically imaging the capillary blood vessels of a living tissue likely to move has been developed to respond at least partially to the above-mentioned drawbacks of the prior art. The device comprises:
- a light source;
- an optical device to guide the light towards the living tissue, the light being absorbed by erythrocytes of the capillary blood vessels;
- a camera for acquiring at least one sequence of images which shows a region of interest of the capillary blood vessels, over a given duration,
- a microscope coupled to the camera,
- a process and display unit connected to the camera, the process and display unit being configured for:
   - selecting images of the sequence, called selected or 'sharp images', arranged in chronological order of acquisition;
   - shuffling the sharp images, for decorrelating temporally the sharp images, by arranging them in a shuffled order different from the chronological order;
   - realigning spatially the sharp images arranged in the shuffled order;
   - generating a projected image by projection of the pixels of the realigned sharp images, in a stack, the projected values of the pixels forming the projected image being:
- extremal intensity values of the pixels of all the sharp images, the projection of the minimal of intensity values of the pixels rendering all the positions of all erythrocytes of all the sharp images in the projected image, or
- average intensity values of the pixels of all the sharp images, the projection of the average intensity values rendering the average flow in the capillary vessels in the projected image;
- or with the maximal of intensity values of the pixels rendering the positions of static erythrocytes i.e. regions of zero flow,
- and preferably displaying the projected image.

As the sharp images are arranged in a shuffled order before being realigned, consecutive shuffled sharp images are less correlated, or not correlated, by the trajectory of cells, as erythrocytes, in the blood vessels. As a consequence, the realignment of the sharp images is possible and/or at least more robust.

In further optional aspects of the invention:
- the process and display unit is configured to project at least some of the sharp images in their chronological order on the projected image presenting average intensity values of the pixels of all the sharp images, to see a video of the flow of erythrocytes in the capillary blood vessels;
- the process and display unit is configured to shuffle the sharp images such that two successive images in the shuffled order correspond to images which are separated one with respect to the other by at least two other images successive in the chronological order, preferably by more than 50 images in the chronological order, preferably corresponding to at least one second in actual time;
- the process and display unit is configured to shuffle the sharp images by separating the sequence of sharp images into at least two subsequences, and by distributing sharp images of the subsequences to define the sharp images of the shuffled order such that successive positions of the shuffled order correspond to sharp images from the different subsequences;
- the process and display unit is configured to select the sharp images among the images of the sequence by calculating the intensity gradient of each image; the unit is configured to select the sharp images among the images of the sequence, by discarding the blurred images and the images which are too different from a chosen reference image in the stack;
- the device comprises a control unit coupled to the camera, the control unit being configured to control the camera to acquire a plurality of sequences, each sequence being acquired during a given time duration, and to control a given length of time between each sequence;
- the process and display unit is configured to calculate the projected image from each respective sequences, the projected images being arranged in the chronological order of acquisition of the sequences, so as to display a video of the evolution ("time-lapse") of the capillary blood vessels with the projected images, at least some of the sharp images being projected in their chronological order on the projected images to see the video of the flow of erythrocytes in the capillary blood vessels;
- the process and display unit is configured to calculate a plurality of projected images in the chronological order of acquisition of the projected images, during several hours or several days, said time duration being less than 1 minute for each sequence and said length of time between two sequences being more than 1 minute;
- the control unit is configured to move the camera if the region of interest moves without returning to its initial position, so as to continue to observe the region of interest after its displacement with respect to the initial position in the living tissue;
- the process and display unit is configured, for selecting the sharp images, to:
   - calculate the minimal reference gray level of the reference image;
   - select the images of the sequence which have a minimal gray level close to the reference gray level by defining a criterion such that a given number of images, for example 50% are considered to be close to the reference image and conserved (this is done by scanning first all images, then selecting the 50% images which are the closest to the reference image)
   - calculate the total intensity by summing all the pixels of the first selected images;
   - select the images among the first selected images which have a total intensity above a threshold, the second selected images being the sharp images (alternatively the second sequence of images which are conserved can be extracted by selecting the first selected images which have a center of gravity of gray levels close to a reference center gravity);
- the process and display unit is configured to calculate the depth of at least a capillary blood vessel from the intensity of the projected image generated by projection of the pixels having an extremal value, so as to deduce the cross section of the capillary blood vessels, in other terms the process and display unit is configured to:
- measure the resulting intensity of all the pixels in the capillary blood vessels on the projected image by the minimal values projection, and
- calculate the depth of the capillary blood vessels perpendicular to the planes of the images; the depth being a function of the intensity of the pixels (which is function of the number of erythrocytes in the Z-direction, which moves in the blood vessels), so as to deduce the cross section of the capillary blood vessels;
- the process and display unit is configured to increase the contrast and/or color of the projected image;
- the spatial realignment comprises shifting spatially and/or rotating each sharp image with respect to the previous sharp image arranged in the shuffled order;
- the camera comprises an image sensor and an optical filter which allows transmission to the image sensor of filtered light at wavelengths comprised between 450 nm and 650 nm, preferably between 490 nm and 590 nm;
- the sequence comprises at least 30 sharp images and preferably at least 100 sharp images for the projected image, the camera being configured to acquire at least 10 images of the sequence per second.

Another aspect of the invention is a computer implemented method for automatically imaging capillary blood vessels of a living tissue likely to move, comprising:
- illuminating the living tissue, the light being absorbed by the erythrocytes of the capillary blood vessels;
- acquiring at least one sequence of images which shows a region of interest of the capillary blood vessels, over a given duration;
- selecting images of the sequence, called selected or 'sharp images', arranged in chronological order of acquisition;
- shuffling the sharp images, for decorrelating temporally the sharp images, by arranging them in a shuffled order different from the chronological order;
- realigning spatially the sharp images arranged in the shuffled order;
- generating a projected image by projection of the pixels of the realigned sharp images, in a stack,
the projected values of the pixels forming the projected image being:
- extremal intensity values of the pixels of all the sharp images, the projection of the extremal of intensity values of the pixels rendering all the positions of all erythrocytes of all the sharp images in the projected image, or
- average intensity values of the pixels of all the sharp images, the projection of the average intensity values rendering the average flow in the capillary vessels in the projected image.

In further optional aspects of the invention:
- after the processing of the projected image, the method comprises the step of projecting at least some of the sharp images in their chronological order on the projected image presenting average intensity values of the pixels of all the sharp images to see a video of the flow of erythrocytes in the capillary blood vessels;
- the shuffling is realized such that two successive images in the shuffled order correspond to images which are separated one with respect to the other by at least two other images successive in the chronological order;
- the shuffling comprises the two following sub-steps: separating the sequence of sharp images into at least two subsequences, and distributing sharp images of the subsequences to define the sharp images of the shuffled order such that successive positions of the shuffled order correspond to sharp images from different subsequences;
- the selection of the sharp images among the images of the sequence is realized by discarding the blurred images;
- the camera is controlled to acquire a plurality of sequences, each sequence being acquired during a given time duration, and to wait for a given duration being between each sequence;
- the method comprises a step of calculating the projected images from the respective sequences, arranged in the chronological order of acquisition of the sequences, one projected image per sequence, so as to display a video of the evolution of the capillary blood vessels with the projected images;
- the method presents the steps of measuring the intensity of the pixels in a capillary blood vessel on the projected image generated by projection of the pixels having an extremal value, and calculating the depth of the capillary blood vessels perpendicular to the planes of the images, the depth being a function of the intensity of the pixels and the width of the capillary blood vessels, so as to deduce the cross section of the capillary blood vessel;
- the capillary blood vessels have width inferior to 100 micrometers;
- the method does not use glue or fluorescence.

Another aspect of the invention is the use of the device for *in vitro* or whenever possible *in vivo,* imaging capillary blood vessels of a living tissue, the living tissue being chosen among:
- the chick chorioallantoic membrane,
- mouse or rat's ear,
- the rabbit mesentery,
- the zebrafish vessels,
- chicken brain,
- chicken retina,
- chicken superficial vasculature of the limb,
- chicken yolk-sac,
- and generally, all embryos developing in ovo (reptiles, birds) and having similar extra-embryonic organs and visible blood vessels (e.g. limb vessels, intersomitic vessels, *etc.*)*,*
- the retina.

Another aspect of the invention is the use of the device for *in vivo* imaging capillary blood vessels of a living tissue, the living tissue being a fragment of a retina of a person.

In a further optional aspect of the invention, the use of the device for *in vivo* imaging capillary blood vessels of a living tissue is combined with the use of a drug or cancer cells to observe the evolution of the capillary blood vessels of a living tissue.

### BRIEF DESCRIPTION OF THE DRAWINGS

The invention will be described by way of example, with reference to the accompanying drawings in which:
- figure 1 illustrates a device according to a possible embodiment of the invention,
- figure 2 illustrates different steps of image processing,
- figure 3, figure 4 and figure 5 are images of a CAM vasculature acquired and processed by the device,
- figure 6 is an image of the terminal arteriole acquired and processed by the device,
- figure 7 is an image of a CAM vasculature acquired and processed by the device presenting hotspots,
- figure 8 is an image of a CAM vasculature acquired and processed by the device,
- figure 9 and figure 10 illustrate terminal segments and proximal segments of arterioles,
- figure 11, figure 12, figure 13 and figure 14 illustrate the optical profile of vessels,
- figure 15 is an image of 3D collaterals acquired and processed by the device,
- figure 16 is an image of the vascular interlace at day 10 acquired and processed by the device,
- figure 17 and figure 18 are images of a CAM vasculature acquired and processed by the device presenting hotspots by selecting minimums or average values of pixels,
- figure 19 is an image of the CAM vasculature acquired by the camera,
- figure 20 is an image of the CAM vasculature acquired and processed by the device,
- figure 21 illustrates a process for measuring the depth of a blood vessel,
- figure 22 is an image of the CAM vasculature acquired and processed by the device.

### DEFINITION

The term "realignment" of images will be used herein to designate the process of transforming different images with a relative motion with respect to each other into one coordinate system of images in which the relative movements are cancelled. The term "realignment" of images is also known as "registering" images.

### DETAILED DESCRIPTION OF PREFERRED ASPECTS OF THE INVENTION

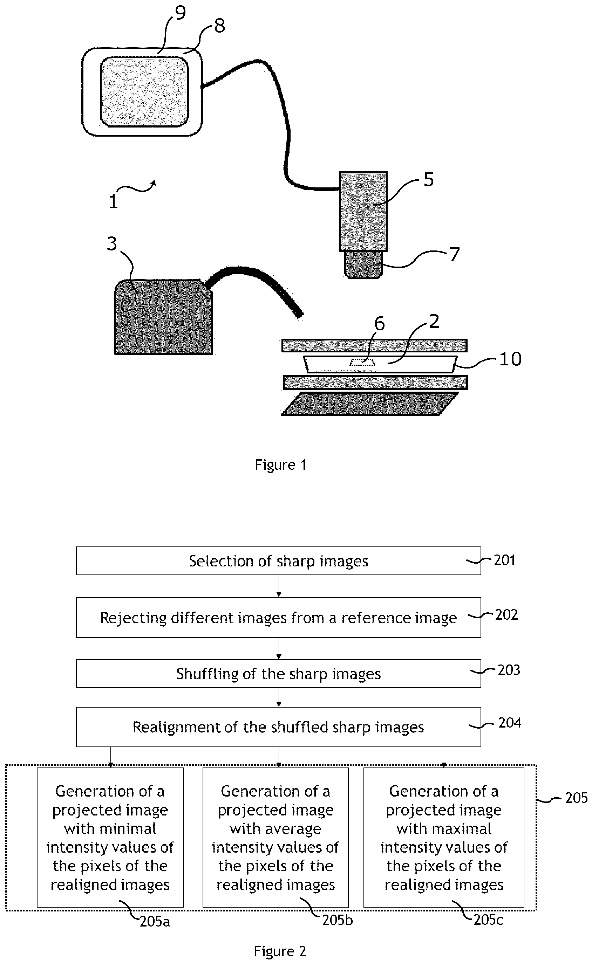

Referring to figure 1, the device 1 is configured for automatically imaging the capillary blood vessels and also larger vessels in the hierarchy of vessels, of a living tissue 2, for example an embryo.

### Embryo

The living tissue 2 can be for example an embryo. Prior to imaging, the embryo is incubated shell less in a plastic cup 10. The plastic cup 10 has a trapezoidal profile, which is important to image the Chorioallantoic Membrane (CAM) vessels by the edge, over a white background. The egg was opened and transferred to the plastic cup in a sterile hood. The shell of the egg was sterilized before being broken. The cup was placed in a large Petri dish (Duroplan 10cm), with optical quality. 3 mm of PBS were poured around the plastic cup inside the Petri dish. The Petri dish comprising the embryo was incubated in an incubator at 37°C. For intra-vital imaging, the embryos, inside the Petri dish, were placed in between two heating stages (Minitüb gmbh). The top heating stage has a central round window.

### Device

The device 1 comprises a light source 3. The light source 3 can be any light source powerful enough (-1500 watts, e.g. fiber lamp Schott). The light is oriented towards the sample by, for example, a light fiber. The light is partially absorbed by the living tissue 2 and especially by the erythrocytes circulating in the capillary blood vessels of the living tissue 2. This will provide the contrast for the image processing.

The device 1 comprises a camera 5 for acquiring at least one sequence of images of a region of interest 6 (ROI) of the capillary blood vessels, over a given duration. The camera 5 can be a Stingray monochrome HD firewire camera from Allied Vision Technology (frame rate 15Hz), or a Basler CMOS monochrome HD USB camera (frame rate 42Hz), or any other camera providing computerized digital images.

The device 1 comprises a microscope 7 coupled to the camera 5. The microscope 7 can be a Leica Macroscope F16 APO. The device 1 also comprises a process and display unit 8 connected to the camera 5/microscope 7, so as the camera 5 can transfer at least an image sequence to the process and display unit 8. The sequence comprises images acquired by the camera 5 in a chronological order.

The microscope 7, the camera 5 and the living tissue 2 are arranged so that the camera 5 can image a region of interest 6 of the living tissue chosen by the user of the device 1.

Preferably, the device 1 comprises an optical filter which permits transmission to the image sensor of filtered light at wavelengths comprised between 450 nm and 650 nm, preferably between 490 nm and 590 nm. Therefore, the imaged erythrocytes correspond to a minimum of measured intensity.

### Process and display unit 8

Referring to figure 2, the process and display unit 8 is configured for performing different steps of image processing.

The living tissue 2, when moving, can indeed be displaced in large proportions, or moved in a direction normal to the plane of observation. The image of the region of interest 6 acquired by the camera 5 can get blurred. Therefore, in a step 201, the process and display unit 8 selects sharp images from the image sequence acquired by the camera 5. Generally, blurred images arise from the oscillatory behavior of the heartbeat of the living tissue and intrinsic movements of the embryo. For example, with proper positioning of the objectives, the region of interest 6 can be acceptably focused for 50% of the time, and out-of-focus 50% of the time, due to the oscillation of the living tissue 2. Approximatively 1 out of 2 plates can then be discarded. When a sharp feature gets out of focus, the light is diffused away, so that typically a sharp dark spot will become lighter in color or in gray level. A crisp and dark area can be selected (automatically or by user) in a reference image of the sequence acquired by the camera 5. The process and display unit 8 is configured to measure the gray level of the area image-by-image and discard for example 50% of the images having a less sharp area, as deduced from measuring the gray level.

The grey level of a selected area can be measured, for example, by calculating the minimal gray level of the selected area. Then, the process and display unit 8 can select first selected images from the sequence, each image of the first selected images having a minimal gray level comprised between the minimal gray level of the reference image and a threshold grey level being higher than the minimal gray level of the reference image. The grey level of a selected area can also be measured by averaging the grey levels of the area. Then, the process and display unit 8 is configured to select the images presenting an average grey level comprised within a given range of grey levels, comprising the average grey level of the reference image.

The sharp images can also be selected by the process and control unit 8 among the images of the sequence by calculating the intensity gradient of each image. Therefore, only the image presenting an intensity gradient superior than a given threshold value can be selected.

The region of interest 6 of the living tissue 2, when moving, can also be displaced out of the field of view seen by the camera 5. Therefore, in a step 202, images which are too different from a chosen reference image in the sequence acquired by the camera 5. The difference between a reference image, chosen by the user, and an image of the sequence can be measured by the same means than in step 201. The process and display unit 8 can then discard some of the images of the sequence for a given threshold grey level.

In a step 203, the images from the output sequence of step 201 and/or of step 202 are shuffled for decorrelating temporally the images, by arranging them in a shuffled order different from the chronological order. The camera 5 can, for example, acquire a sequence comprising between 50 and 300 images (for example at 15hz) which amounts to 5 to 20 seconds of video acquisition. After step 203, two successive images in the shuffled order correspond to images which are separated one with respect to the other by at least two other images successive in the chronological order, notably by more than 10 images and preferably by more than 25 images in the chronological order, preferably corresponding to at least one second in actual time.

The process and display unit 8 can be configured for separating the output sequence of steps 201 and/or of step 202 in two sub-sequences and distributing sharp images of the subsequences to define the sharp images of the shuffled order such that successive positions of the shuffled order correspond to sharp images from the different subsequences.

In order to do so, the process and display unit 8 can, for example, separate the output sequence of step 201 and/or 202 in a first subsequence and a second subsequence of equal number of images (in the case of an even number of images in the output sequence of step 201 and/or 202). For Nᵢₘ being the number of images in the sequence, k being an integer, the process and display unit 8 can be configurated for shuffling the positions of each 2k+1 image between 1 and Nᵢₘ/2 of the first subsequence respectively by each Nᵢₘ/2+*k* image of the second subsequence. Therefore, the temporal decorrelation between the images of the sequence can be maximized.

Alternatively, the images of the output sequence of step 201 and/or 202 can be randomly shuffled.

In a step 204, the images arranged in the shuffled order, *i.e.* the images of the output sequence of step 203, are realigned (*i.e.* are registrated). The output sequence of step 203 is for example aligned using the plugin StackReg (Biomedical Imaging Group, EPFL) in the software ImageJ. Each image is used as the template with respect to which the next image is aligned, so that the alignment proceeds by propagation. The user can select the reference image from which the sequence is aligned. Each image of the sequence can be aligned towards the previous image by translation, by rigid body transformation, by scaled rotation and/or by affine transformation.

The realignment of the step 204 is made possible by the step 203 of shuffling. The region of interest 6 presents two types of movements: the background movement of the living tissue 2 and the movement of the flowing erythrocytes in the blood vessels or capillaries. If realigning a sequence without shuffling the different images, the realignment process can consider the two types of movement between two consecutive images, and therefore align considering the movement of the erythrocytes. By shuffling the images of the sequence in step 203, the movement of the erythrocytes is not detectable between two consecutive images, and therefore, the images are aligned considering only the background movement of the living tissue 2.

In a step 205, the process and display unit 8 is configured to generate a projected image by projection of the pixels of the realigned sharp images, *i.e.* the image of the output sequence of step 204.

In a step 205a, the projected values of the pixels of the projected image are minimum intensity values of the pixels of all the sharp images, *i.e.* of the images of the output sequence of step 204. Preferably, the extremal intensity values of the pixels are minimum intensity values. Therefore, the projection is extracting each point in the sequence where at least one erythrocyte (for example a black dot) has passed once during the time of acquisition by the camera 5. All the positions of all erythrocytes of all the sharp images are rendered in the projected image. The most visible erythrocyte is kept for generating the image. As a result, the "minima image" (*i.e.* the output image of step 205a) presents the lumens of the capillary blood vessels, as if imaged by a homogeneous light from the inside the blood vessel. The output sequence of step 204 can be projected by using the function "Zproject" from the software ImageJ, and by choosing the option "minimum intensity". As an alternative, the process can have a step of image value inversion before step 205, and in step 205a, the projected values of the pixels of the projected image are maximum intensity values of the pixels of all the sharp images.

In a step 205b, the projected values of the pixels of the projected image are the average intensity values of the pixels of all the sharp images, *i.e.* of the images of the output sequence of step 204. In step 205b, the average red blood cell count passed at each point over time is measured. Therefore, the output image after step 205b corresponds to the flowrate of erythrocytes.

In a step 205c, the projected values of the pixels of the projected image are the maximal intensity values of the pixels of all the sharp images, *i.e.* of the images of the output sequence of step 204. In step 205c, the maximal value returns a "white pixel" if the erythrocytes have moved in the region of this pixel since there will be at least one image in which the pixel will be transiently white, this white level representing the light level of the plasma between two flowing erythrocytes. Conversely, if an erythrocyte is always static at the position of this pixel, the maximal value will be equal to the "black" level of this erythrocyte, since, being immobile, it will be present in all images. Therefore, the output image after step 205c corresponds to zones where the flow is stagnant (zero flow).

Therefore, the imaging of the erythrocyte flow in capillary blood vessels by the device 1 is cheap, does not require fluorescent labelling of cells, does not require injection of fluorescent dies such as FITC dextran and does not require fixation of the living tissue 2. Moreover, the magnitudes of the flow can be measured qualitatively and quantitatively. The image is formed from sources dispersed inside the vessel lumen: therefore, a volume rendering of the vascular surface profile can be imaged by the device 1. Images are obtained by following individual erythrocytes passing over a homogeneous white field. The resolution of the method is for example good enough to provide images of capillaries at magnifications 1X to 9X with a binocular.

Preferably, imaging can be time lapsed. The device 1 can comprise a control unit 9 coupled to the camera 5. The control unit 9 is configured to control the camera so as to acquire a plurality of sequences along time. An image can be obtained from each sequence, following at least the steps 201, 202, 203, 204 and 205a and/or 201, 202, 203, 204 and 205b or 205c. A video of the obtained images can be made, so as to display the evolution of the capillary blood vessels. Preferably, each sequence is acquired during a given time duration, and the control unit 9 is configured such as a given length of time separates each sequence. Notably, the time lapse recording can last for hours or days, while recording can last preferably less than 1 minute. Each recording of a sequence of images can, for example, be separated by a time length of 5 minutes.

### Examples

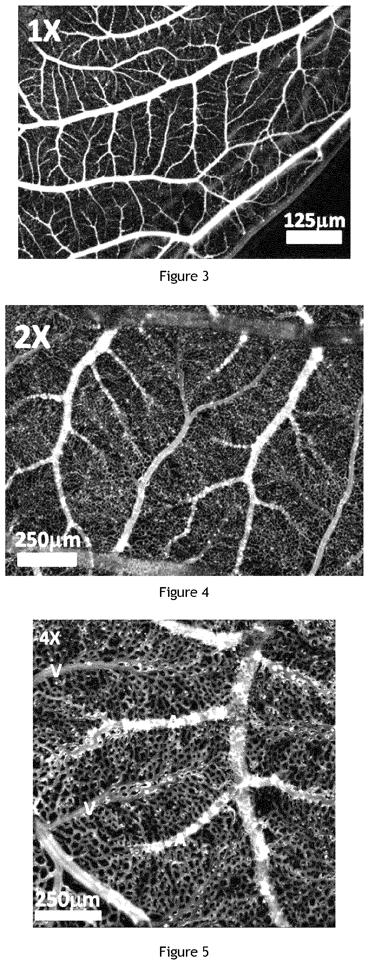

Referring to figure 3, figure 4 and figure 5, images of a CAM vasculature at day 10 of development of the embryo, are obtained by the device 1. The images are taken with a low resolution binocular, allowing imaging in wide field (1X in figure 3) but also at higher level of magnification (2X in figure 4 and 4X in figure 5). Figure 3, figure 4 and figure 5 illustrate the interlace structure of the capillaries.

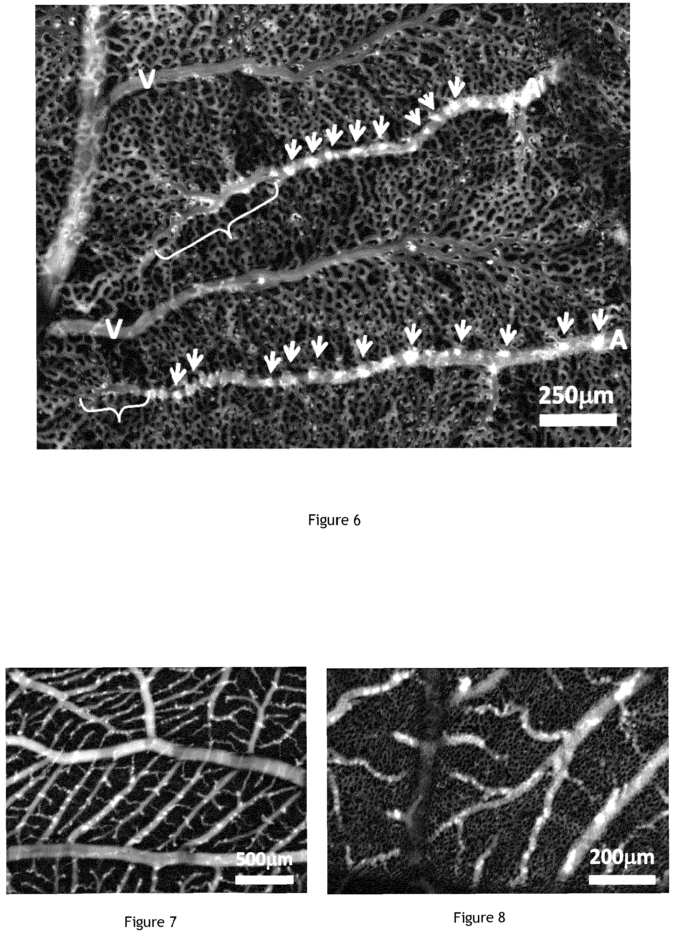

Referring to figure 6, the terminal arterioles can be imaged by the device 1. Vertical chimneys can be observed (illustrated by arrows in figure 6), regularly spaced along the arterioles, from which the flow is higher (and not along veins). These chimneys are visible by hotspots corresponding to erythrocytes flowing towards the plane of view of figure 6. They appear bright because of the stacking effect of the red cells which increases the contrast. However, as one approaches the distal end of the arteriole, there exists a flat area devoid of vertical chimneys (illustrated by the brackets in figure 6).

Referring to figure 7, hotspots point can be visible, for example up to the 14^{th} day of development of the embryo. The hotspots points are indicated by the white arrows. The hotspot points correspond to capillaries presenting a direction out of the plane of observation of figure 7. As the CAM develops, the interlace becomes thinner, with smaller capillary anastomoses.

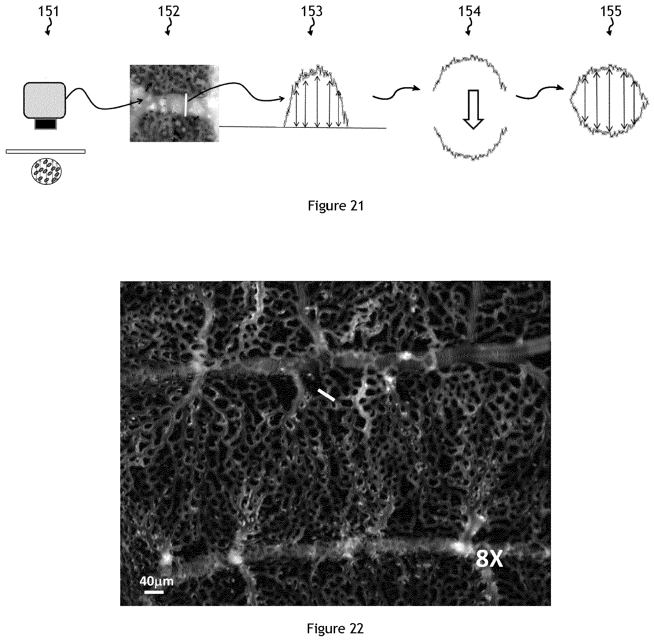

Referring to figure 21, capillaries of very small size, for example having a diameter smaller than 8 micrometers can be imaged with the device 1.

Despite ample embryo movements at later stages (14^{th} day of development of the embryo, figure 8), the capillary lattice can be imaged by the device 1, and the vessels at the upper levels of the hierarchy can also be imaged by filming the embryo at the periphery of the plastic cup 10 where movements are smaller, or by carefully selecting a favorable area elsewhere.

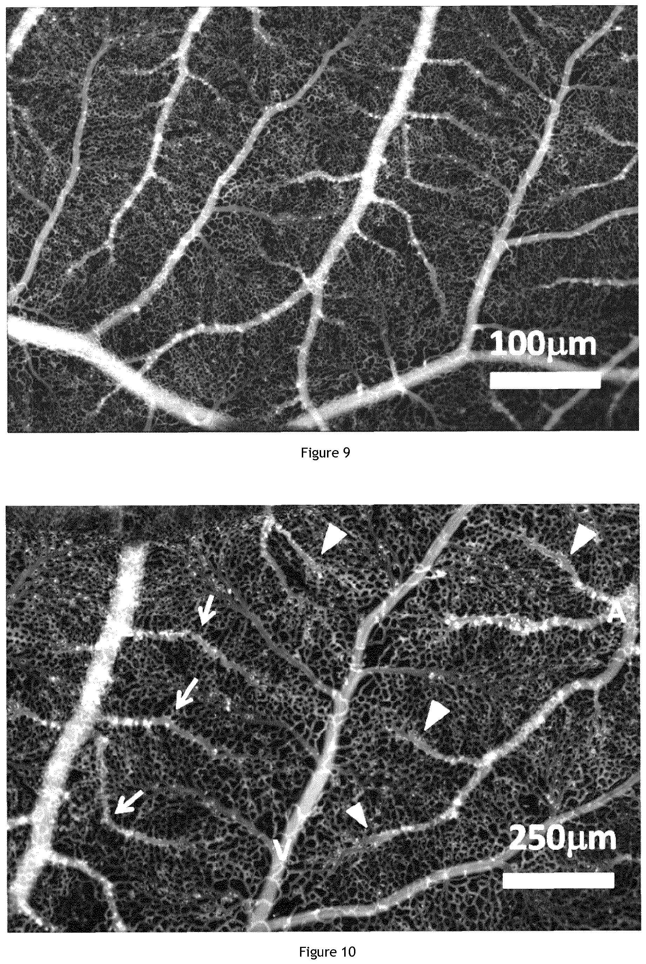

Referring to figure 9 and to figure 10, arterioles exhibit a terminal segment which is distinctly different from the proximal segment. The arterial pattern shows the presence of vertical "chimneys" or "springs" of flow identified by the presence of hotspots along the arterioles. The hotspots provide flow sources located proximally with respect to the arteriolar hierarchy. The veins do not navigate towards the tip of arteries where the flat distal areas are found (illustrated by arrows in figure 10). Referring to figure 10, a magnification shows that the flat distal areas are wider than the more proximal part of the arteries. The distal segment often has a "goose leg" widening (illustrated in figure 10 by arrowheads).

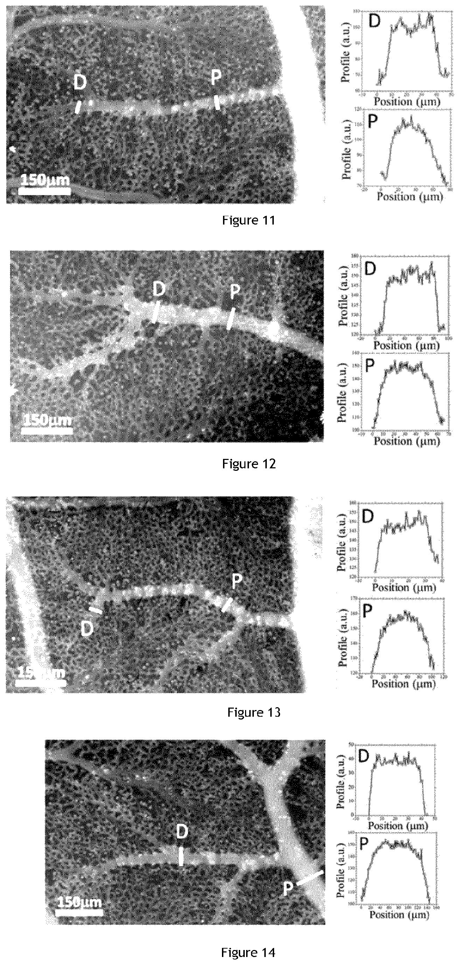

Referring to figure 11, figure 12, figure 13 and figure 14, the optical profile of vessels can be calculated and plotted from an image of the device 1. The terminal parts of the arterioles appear flat (graphs D in figure 11, figure 12, figure 13 and figure 14), while more proximal vessels appear round (graphs D in figure 11, figure 12, figure 13 and figure 14). The white lines correspond to the locations where the profiles were extracted, D stands for Distal and P for Proximal. This shows that the terminal part of the arteriole is flattened on the underneath surface of the ectoderm on which it creeps (N=30 arterioles were processed giving similar results).

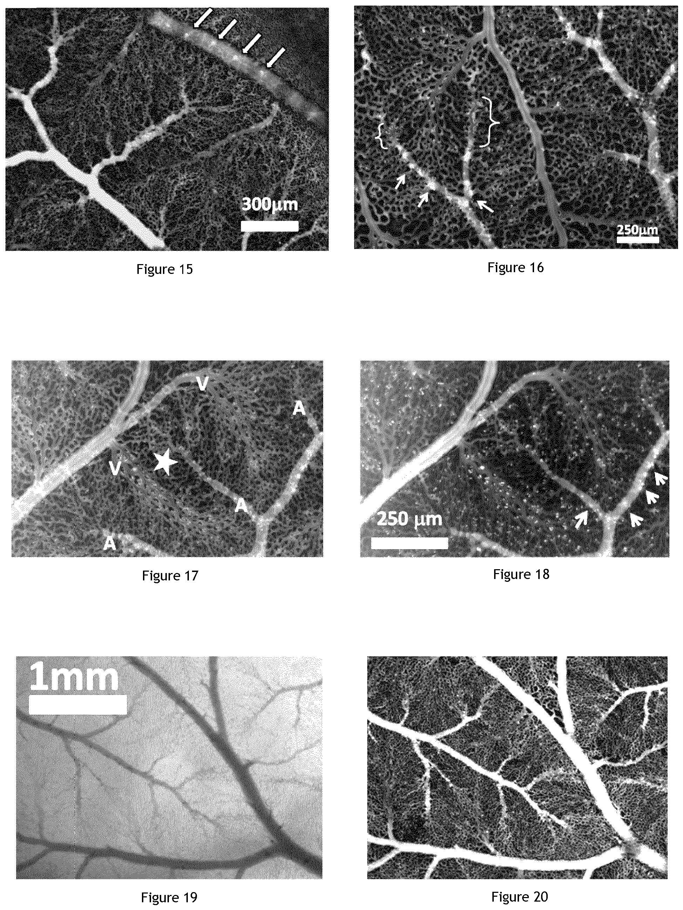

Referring to figure 15, the device 1 can image 3D collaterals pointing upwards attracting veins. The vertical chimneys along the arterioles serve as flow sources attracting hemodynamically the veins proximally. Figure 15 corresponds to a day 7 embryo, in which the vessels are quite coarse.

Figure 16 corresponds to the vascular interlace at day 10. The capillary plexus is oriented at day 10 along the path starting at the vertical chimneys and flowing towards the presumptive vein. Careful inspection allows one to distinguish the swollen area under the distal flattened segment of the arteriole. The brackets show the flat distal segments. A dimmer area corresponding to the flattened plexus is imaged around the two segments close to the brackets.

Figure 17 illustrates an image acquired by the device 1, corresponding to the output of step 205a, wherein the generation of projected image is processed with minimal intensity values of the pixels of the realigned image.

Figure 18 illustrates an image acquired by the device 1, corresponding to the output of step 205b, wherein the generation of projected image is processed with average intensity values of the pixels of the realigned image. An area presenting a lower flowrate is illustrated by a star in figure 17, along the "flat" distal part of the vessel. The veins develop towards the vertical chimneys located more proximally along the arteries, illustrated by white arrows. The device 1 images typical interdigitating artery and veins, in both average mode and minimal intensity mode.

Figure 19 illustrates an image of the input sequence acquired by the camera 5 before step 201. In comparison, figure 20 illustrates an image obtained by the device 1 after step 205a, corresponding to the same field of view than in figure 19.

Figure 20 illustrates an image of the input sequence at the magnification x8, showing a scale bar of 40 micrometers, and the typical dimension of capillaries at this stage, around and below 8 micrometers.

In reference to figure 21, the depth of at least a capillary blood vessel can be calculated from the intensity of the projected image generated by projection of the pixels having an extremal value in step 205a, so as to deduce the cross section of the capillary blood vessels. In a step 151, the projected image of step 205b is calculated, for example from the acquisition of 300 images at 15Hz. The projected image is inverted to get a gray level view of the absorption. In step 152, a section is chosen, illustrated by a white line in figure 14. The absorption across the vessel in the vertical direction is proportional to local vessel depth. In step 153, a profile of absorption perpendicularly to the vessel is extracted from an intensity measurement of the projected image, at some straight location in between collaterals where a tubular segment is found. The profile of absorption has a height proportional to vessel depth. In a step 154, the measured halve is symmetrized. In a step 155, the vessel cross section is reconstructed by assembling both halves, by assuming a bilateral symmetry. Larger vessels which have higher pressure and are obviously almost cylindrical can be used for calibration. We assume in this set up that the optical refraction index of the plasma is identical to the optical refraction index of the tissue. This amounts to neglecting the cylindrical aberration due to the cylinder/plan diopter. For adjusted indices, the optical path across a cylinder embedded in an image behaves as for a flat diopter, and there is no refractive distortion.

Figure 22 is an image of the CAM vasculature acquired and processed by the device. The scale bar corresponds to 40 µm. The image is acquired at a magnification x8.

## Claims

1. A device (1) for automatically imaging the capillary blood vessels of a living tissue (2) likely to move, comprising:
• a light source (3);
• an optical device (4) to guide the light towards the living tissue (2), the light being absorbed by erythrocytes of the capillary blood vessels;
• a camera (5) for acquiring at least one sequence of images which shows a region of interest (6) of the capillary blood vessels, over a given duration, the absorption of the light by the erythrocytes on the images showing the capillary blood vessels,
• a microscope (7) connected to the camera (5),
• a process and display unit (8), connected to the camera (5),
• the process and display unit being configured for:
- selecting images of the sequence, called 'selected images', arranged in chronological order of acquisition;
- shuffling the selected images, for decorrelating temporally the selected images, by arranging them in a shuffled order different from the chronological order;
- realigning spatially the selected images arranged in the shuffled order;
- generating a projected image by projection of the pixels of the realigned selected images, in a stack,
the projected values of the pixels forming the projected image being:
• minimal intensity values of the pixels of all the selected images, the projection of the minimal of intensity values of the pixels rendering all the positions of all erythrocytes of all the selected images in the projected image, or
• average intensity values of the pixels of all the selected images, the projection of the average intensity values rendering the average flow in the capillary vessels in the projected image, or
• maximal intensity values of the pixels of all the selected images, the projection of the maximal of intensity values of the pixels rendering the positions where the flow of erythrocytes is stagnant.

2. The device according to claim 1, wherein the process and display unit (8) is configured for re-aligning spatially the selected images in the shuffled order, without taking into account a fixed common vascular pattern of all the selected images.

3. The device according to claims 1-2, wherein the process and display unit (8) is configured to project at least some of the selected images in their chronological order on the projected image to see a video of the flow of erythrocytes in the capillary blood vessels.

4. The device according to claims 1-3, wherein the process and display unit (8) is configured to shuffle the selected images such that two successive images in the shuffled order correspond to images which are separated one with respect to the other by at least two other images successive in the chronological order, preferably by more than 50 images in the chronological order, preferably corresponding to at least one second in actual time.

5. The device according to any of claims 1 to 4, wherein the process and display unit (8) is configured to shuffle the selected images by:
- separating the sequence of selected images into at least two subsequences,
- distributing selected images of the subsequences to define the selected images of the shuffled order such that successive positions of the shuffled order correspond to selected images from the different subsequences.

6. The device according to any of claims 1 to 5, wherein the device (1) comprises a control unit (9) coupled to the camera (5), the control unit (9) being configured to control the camera (8) to acquire a plurality of sequences, each sequence being acquired during a given time duration, and to control a given length of time between each sequence.

7. The device according to claim 6, wherein the process and display unit (8) is configured to calculate the projected images from the respective sequences, arranged in the chronological order of acquisition of the sequences, one projected image per sequence, so as to display a video of the evolution of the capillary blood vessels with the projected images at least some of the selected images being projected in their chronological order on the projected images to see the video of the flow of erythrocytes in the capillary blood vessels.

8. The device according to any of claims 6 to 7, wherein the control unit (9) is configured to move the camera if the region of interest moves without returning to its initial position, so as to continue to observe the region of interest after its displacement with respect to the initial position in the living tissue.

9. Device according to claims 1-8, wherein, for obtaining the selected images, the process and display unit (8) is configured to:
- calculate the minimal reference gray level of the reference image;
- select the images of the sequence which have a minimal gray level close to the reference gray level by defining a criterion such that a given number of images, for example 50% are considered to be close to the reference image and conserved;
- calculate the total intensity by summing all the pixels of the first selected images;
- select the images among the first selected images which have a total intensity above a threshold, the second selected images selected being the selected images.

10. The device according to any of claims 1 to 9, wherein the process and display unit (8) is configured to:
- measure the resulting intensity of the all pixels in the capillary blood vessels on the projected image by the minimal values projection, and
- calculate the depth of the capillary blood vessels perpendicular to the planes of the images;
the depth being a function of the intensity of the pixels (which is function of the number of erythrocytes in the Z-direction, which moves in the blood vessels) so as to deduce the cross section of the capillary blood vessels.

11. The device according to any of claims 1 to 10, wherein the spatial realignment comprises shifting spatially and/or rotating each selected image with respect to the previous selected image arranged in the shuffled order.

12. The device according to any of claims 1 to 11, wherein the camera comprises an image sensor and an optical filter which allows transmission to the image sensor of filtered light at wavelengths comprised between 450 nm and 650 nm, preferably between 490 nm and 590 nm.

13. The device according to any of claims 1 to 12, wherein the sequence comprises at least 30 selected images and preferably at least 100 selected images for the projected image, the camera being configured to acquire at least 10 images of the sequence per second.

14. Computer implemented method for automatically imaging capillary blood vessels of a living tissue (2) likely to move, comprising:
- illuminating the living tissue, the light being partially absorbed by the erythrocytes of the capillary blood vessels;
- acquiring at least one sequence of images which shows a region of interest (6) of the capillary blood vessels, over a given duration,
- selecting images of the sequence, called 'selected images', arranged in chronological order of acquisition;
- shuffling the selected images, for decorrelating temporally the selected images, by arranging them in a shuffled order different from the chronological order;
- realigning spatially the selected images arranged in the shuffled order;
- generating a projected image by projection of the pixels of the realigned selected images, in a stack,
the projected values of the pixels forming the projected image being:
• minimal intensity values of the pixels of all the selected images, the projection of the minimal of intensity values of the pixels rendering all the positions of all erythrocytes of all the selected images in the projected image, or
• average intensity values of the pixels of all the selected images, the projection of the average intensity values rendering the average flow in the capillary vessels in the projected image or
• maximal intensity values of the pixels of all the selected images, the projection of the maximal of intensity values of the pixels rendering the positions where the flow is stagnant

15. The method according to claim 14, wherein the spatial re-alignment of the selected images in the shuffled order, is realized without taking into account a fixed common vascular pattern of all the selected images.

16. The method according to claims 14-15, wherein after the processing of the projected image, the method comprises the step of projecting at least some of the selected images in their chronological order on the projected image to see a video of the flow of erythrocytes in the capillary blood vessels.

17. The method according to any of claims 15-16, wherein the shuffling comprises the two following sub-steps:
- separating the sequence of selected images into at least two subsequences,
- distributing selected images of the subsequences to define the selected images of the shuffled order such that successive positions of the shuffled order correspond to selected images from different subsequences.

18. The method according to claims 15-17, wherein the camera (8) is controlled to acquire a plurality of sequences, each sequence being acquired during a given duration, and to wait for a given length of time between each sequence,
the method comprises a step of calculating the projected images from the respective sequences, arranged in the chronological order of acquisition of the sequences, one projected image per sequence, so as to display a video of the evolution of the capillary blood vessels with the projected images.

19. The method according to any of claims 15 to 18, wherein the method comprises the steps of:
- measuring the intensity of the pixels in a capillary blood vessel on the projected image, and
- calculating the depth of the capillary blood vessels perpendicular to the planes of the images;
the depth being a function of:
- the intensity of the pixels and
- the width of the capillary blood vessels,
so as to deduce the cross section of the capillary blood vessel.

20. Use of a device according to any of claims 1 to 13 for *in vitro*/*in vivo* imaging capillary blood vessels of a living tissue,
the living tissue being chosen among:
- the chick chorioallantoic membrane,
- mouse or rat's ear,
- the rabbit mesentery,
- the zebrafish vessels,
- chicken brain,
- chicken retina,
- chicken superficial vasculature of the limb,
- chicken yolk-sac,
- and generally, all embryos developing in ovo (reptiles, birds) and having similar extra-embryonic organs and visible blood vessels (e.g. limb vessels, intersomitic vessels etc.),
- the retina.

## Patentansprüche

1. Vorrichtung (1) zur automatischen Bildgebung der kapillaren Blutgefäße eines lebenden Gewebes (2), das sich wahrscheinlich bewegen wird, umfassend:
• eine Lichtquelle (3);
• eine optische Vorrichtung (4), um das Licht auf das lebende Gewebe (2) zu lenken, wobei das Licht von Erythrozyten der kapillaren Blutgefäße absorbiert wird;
• eine Kamera (5), um über eine gegebene Dauer mindestens eine Bildsequenz aufzunehmen, die einen interessierenden Bereich (6) der kapillaren Blutgefäße zeigt, wobei die Absorption des Lichts durch die Erythrozyten in den Bildern die kapillaren Blutgefäße zeigt,
• ein Mikroskop (7), das mit der Kamera (5) verbunden ist,
• eine Verarbeitungs- und Anzeigeeinheit (8), die mit der Kamera (5) verbunden ist,
• wobei die Verarbeitungs- und Anzeigeeinheit dafür ausgelegt ist:
- Bilder aus der Sequenz auszuwählen, die als "ausgewählte Bilder" bezeichnet werden und in chronologischer Reihenfolge der Aufnahme angeordnet sind;
- die ausgewählten Bilder zu mischen, um die ausgewählten Bilder zeitlich zu dekorrelieren, indem sie in einer gemischten Reihenfolge angeordnet werden, die sich von der chronologischen Reihenfolge unterscheidet;
- die in der gemischten Reihenfolge angeordneten ausgewählten Bilder räumlich neu auszurichten;
- durch Projektion der Pixel der neu ausgerichteten ausgewählten Bilder in einem Stapel ein projiziertes Bild zu erzeugen,
wobei es sich bei den projizierten Werten der Pixel, die das projizierte Bild bilden, handelt um:
• minimale Intensitätswerte der Pixel aller ausgewählten Bilder, wobei die Projektion des Minimums von Intensitätswerten der Pixel im projizierten Bild alle Positionen aller Erythrozyten aller ausgewählten Bilder wiedergibt, oder
• durchschnittliche Intensitätswerte der Pixel aller ausgewählten Bilder, wobei die Projektion der durchschnittlichen Intensitätswerte im projizierten Bild die durchschnittliche Strömung in den Kapillargefäßen wiedergibt, oder
• maximale Intensitätswerte der Pixel aller ausgewählten Bilder, wobei die Projektion des Maximums von Intensitätswerten der Pixel die Positionen wiedergibt, an denen die Erythrozytenströmung stagniert.

2. Vorrichtung nach Anspruch 1, wobei die Verarbeitungs- und Anzeigeeinheit (8) dafür ausgelegt ist, die ausgewählten Bilder in der gemischten Reihenfolge räumlich neu auszurichten, ohne ein festes gemeinsames Gefäßmuster aller ausgewählten Bilder zu berücksichtigen.

3. Vorrichtung nach den Ansprüchen 1-2, wobei die Verarbeitungs- und Anzeigeeinheit (8) dazu ausgelegt ist, im projizierten Bild mindestens einige der ausgewählten Bilder in ihrer chronologischen Reihenfolge zu projizieren, um ein Video der Erythrozytenströmung in den kapillaren Blutgefäßen zu sehen.

4. Vorrichtung nach den Ansprüchen 1-3, wobei die Verarbeitungs- und Anzeigeeinheit (8) dazu ausgelegt ist, die ausgewählten Bilder so zu mischen, dass zwei aufeinanderfolgende Bilder in der gemischten Reihenfolge Bildern entsprechen, die in Bezug zueinander um mindestens zwei andere Bilder, die in der chronologischen Reihenfolge aufeinanderfolgen, vorzugsweise um mehr als 50 Bilder in der chronologischen Reihenfolge, die vorzugsweise mindestens einer Sekunde in der tatsächlichen Zeit entsprechen, getrennt sind.

5. Vorrichtung nach einem der Ansprüche 1 bis 4, wobei die Verarbeitungs- und Anzeigeeinheit (8) dazu ausgelegt ist, die ausgewählten Bilder zu mischen durch:
- Aufteilen der Sequenz ausgewählter Bilder in mindestens zwei Teilsequenzen,
- Verteilen ausgewählter Bilder der Teilsequenzen, um die ausgewählten Bilder der gemischten Reihenfolge so zu definieren, dass aufeinanderfolgende Positionen der gemischten Reihenfolge ausgewählten Bildern aus den verschiedenen Teilsequenzen entsprechen.

6. Vorrichtung nach einem der Ansprüche 1 bis 5, wobei die Vorrichtung (1) eine Steuereinheit (9) umfasst, die mit der Kamera (5) gekoppelt ist, wobei die Steuereinheit (9) dazu ausgelegt ist, die Kamera (8) zu steuern, um eine Vielzahl von Sequenzen aufzunehmen, wobei jede Sequenz während einer gegebenen Zeitdauer aufgenommen wird, und eine gegebene Zeitlänge zwischen jeder Sequenz zu steuern.

7. Vorrichtung nach Anspruch 6, wobei die Verarbeitungs- und Anzeigeeinheit (8) dazu ausgelegt ist, die projizierten Bilder anhand der jeweiligen Sequenzen, die in der chronologischen Aufnahmereihenfolge der Sequenzen angeordnet sind, ein projiziertes Bild pro Sequenz, zu berechnen, um mit den projizierten Bildern ein Video der Entwicklung der kapillaren Blutgefäße anzuzeigen, wobei mindestens einige der ausgewählten Bilder in den projizierten Bildern in ihrer chronologischen Reihenfolge projiziert werden, um das Video der Erythrozytenströmung in den kapillaren Blutgefäßen zu sehen.

8. Vorrichtung nach einem der Ansprüche 6 bis 7, wobei die Steuereinheit (9) dazu ausgelegt ist, die Kamera zu bewegen, wenn sich der interessierende Bereich bewegt, ohne in seine Ausgangsposition zurückzukehren, um den interessierenden Bereich nach seiner Verlagerung in Bezug auf die Ausgangsposition im lebenden Gewebe weiterhin zu beobachten.

9. Vorrichtung nach den Ansprüchen 1-8, wobei die Verarbeitungs- und Anzeigeeinheit (8), um die ausgewählten Bilder zu erhalten, dazu ausgelegt ist:
- die minimale Referenzgraustufe des Referenzbildes zu berechnen;
- die Bilder der Sequenz, die eine minimale Graustufe nahe der Referenzgraustufe aufweisen, auszuwählen, indem ein Kriterium so definiert wird, dass eine gegebene Anzahl von Bildern, zum Beispiel 50 %, als dem Referenzbild nahe erachtet und aufbewahrt werden;
- die Gesamtintensität zu berechnen, indem alle Pixel der ersten ausgewählten Bilder summiert werden;
- die Bilder unter den ersten ausgewählten Bildern auszuwählen, die eine Gesamtintensität oberhalb einer Schwelle aufweisen, wobei es sich bei den zweiten ausgewählten Bildern, die ausgewählt werden, um die ausgewählten Bilder handelt.

10. Vorrichtung nach einem der Ansprüche 1 bis 9, wobei die Verarbeitungs- und Anzeigeeinheit (8) dazu ausgelegt ist:
- die resultierende Intensität aller Pixel in den kapillaren Blutgefäßen im projizierten Bild durch die Projektion der minimalen Werte zu messen, und
- die Tiefe der kapillaren Blutgefäße senkrecht zu den Ebenen der Bilder zu berechnen;
wobei die Tiefe von der Intensität der Pixel abhängt (die von der sich in den Blutgefäßen bewegenden Anzahl von Erythrozyten in der Z-Richtung abhängt),
um den Querschnitt der kapillaren Blutgefäße abzuleiten.

11. Vorrichtung nach einem der Ansprüche 1 bis 10, wobei die räumliche Neuausrichtung das räumliche Verschieben und/oder Drehen jedes ausgewählten Bildes in Bezug auf das vorhergehende ausgewählte Bild, das in der gemischten Reihenfolge angeordnet ist, umfasst.

12. Vorrichtung nach einem der Ansprüche 1 bis 11, wobei die Kamera einen Bildsensor und einen optischen Filter umfasst, der das Übertragen von gefiltertem Licht mit Wellenlängen im Bereich zwischen 450 nm und 650 nm, vorzugsweise zwischen 490 nm und 590 nm, zum Bildsensor ermöglicht.

13. Vorrichtung nach einem der Ansprüche 1 bis 12, wobei die Sequenz mindestens 30 ausgewählte Bilder und vorzugsweise mindestens 100 ausgewählte Bilder für das projizierte Bild umfasst, wobei die Kamera dazu ausgelegt ist, pro Sekunde mindestens 10 Bilder der Sequenz aufzunehmen.

14. Computerimplementiertes Verfahren zur automatischen Bildgebung von kapillaren Blutgefäßen eines lebenden Gewebes (2), das sich wahrscheinlich bewegen wird, umfassend:
- Beleuchten des lebenden Gewebes, wobei das Licht teilweise von den Erythrozyten der kapillaren Blutgefäße absorbiert wird;
- Aufnehmen mindestens einer Bildsequenz, die einen interessierenden Bereich (6) der kapillaren Blutgefäße zeigt, über eine gegebene Dauer,
- Auswählen von Bildern der Sequenz, die als "ausgewählte Bilder" bezeichnet werden und in chronologischer Reihenfolge der Aufnahme angeordnet sind;
- Mischen der ausgewählten Bilder, um die ausgewählten Bilder zeitlich zu dekorrelieren, indem sie in einer gemischten Reihenfolge angeordnet werden, die sich von der chronologischen Reihenfolge unterscheidet;
- räumliches Neuausrichten der ausgewählten Bilder, die in der gemischten Reihenfolge angeordnet sind;
- Erzeugen eines projizierten Bildes durch Projektion der Pixel der neu ausgerichteten ausgewählten Bilder in einem Stapel,
wobei es sich bei den projizierten Werten der Pixel, die das projizierte Bild bilden, handelt um:
• minimale Intensitätswerte der Pixel aller ausgewählten Bilder, wobei die Projektion des Minimums von Intensitätswerten der Pixel im projizierten Bild alle Positionen aller Erythrozyten aller ausgewählten Bilder wiedergibt, oder
• durchschnittliche Intensitätswerte der Pixel aller ausgewählten Bilder, wobei die Projektion der durchschnittlichen Intensitätswerte im projizierten Bild die durchschnittliche Strömung in den Kapillargefäßen wiedergibt, oder
• maximale Intensitätswerte der Pixel aller ausgewählten Bilder, wobei die Projektion des Maximums von Intensitätswerten der Pixel die Positionen wiedergibt, an denen die Strömung stagniert.

15. Verfahren nach Anspruch 14, wobei die räumliche Neuausrichtung der ausgewählten Bilder in der gemischten Reihenfolge ausgeführt wird, ohne ein festes gemeinsames Gefäßmuster aller ausgewählten Bilder zu berücksichtigen.

16. Verfahren nach den Ansprüchen 14-15, wobei das Verfahren nach dem Verarbeiten des projizierten Bildes den Schritt des Projizierens mindestens einiger der ausgewählten Bilder in ihrer chronologischen Reihenfolge im projizierten Bild umfasst, um ein Video der Erythrozytenströmung in den kapillaren Blutgefäßen zu sehen.

17. Verfahren nach einem der Ansprüche 15-16, wobei das Mischen die zwei folgenden Teilschritte umfasst:
- Aufteilen der Sequenz ausgewählter Bilder in mindestens zwei Teilsequenzen,
- Verteilen ausgewählter Bilder der Teilsequenzen, um die ausgewählten Bilder der gemischten Reihenfolge so zu definieren, dass aufeinanderfolgende Positionen der gemischten Reihenfolge ausgewählten Bildern aus verschiedenen Teilsequenzen entsprechen.

18. Verfahren nach den Ansprüchen 15-17, wobei die Kamera (8) gesteuert wird, um eine Vielzahl von Sequenzen aufzunehmen, wobei jede Sequenz während einer gegebenen Dauer aufgenommen wird, und zwischen jeder Sequenz eine gegebene Zeitlänge zu warten,
das Verfahren einen Schritt des Berechnens der projizierten Bilder anhand der jeweiligen Sequenzen, die in der chronologischen Aufnahmereihenfolge der Sequenzen angeordnet sind, ein projiziertes Bild pro Sequenz, umfasst, um mit den projizierten Bildern ein Video der Entwicklung der kapillaren Blutgefäße anzuzeigen.

19. Verfahren nach einem der Ansprüche 15 bis 18, wobei das Verfahren die Schritte umfasst des:
- Messens der Intensität der Pixel in einem kapillaren Blutgefäß im projizierten Bild, und
- Berechnens der Tiefe der kapillaren Blutgefäße senkrecht zu den Ebenen der Bilder;
wobei die Tiefe abhängig ist von:
- der Intensität der Pixel, und
- der Breite der kapillaren Blutgefäße,
um den Querschnitt des kapillaren Blutgefäßes abzuleiten.

20. Verwendung einer Vorrichtung nach einem der Ansprüche 1 bis 13 zur *in-vitro-*/*in-vivo-*Bildgebung von kapillaren Blutgefäßen eines lebenden Gewebes,
wobei das lebende Gewebe ausgewählt ist aus:
- der Chorioallantoismembran von Küken,
- dem Ohr von Mäusen oder Ratten,
- dem Mesenterium von Kaninchen,
- den Gefäßen von Zebrafischen,
- dem Gehirn von Hühnern,
- der Netzhaut von Hühnern,
- dem oberflächlichen Gefäßsystem der Gliedmaße von Hühnern,
- dem Dottersack von Hühnern,
- und allgemein allen Embryos, die sich in ovo entwickeln (Reptilien, Vögel) und ähnliche extraembryonale Organe und sichtbare Blutgefäße (z. B. Gliedmaßengefäße, intersomitische Gefäße usw.) aufweisen,
- der Netzhaut.

## Revendications

1. Dispositif (1) pour automatiquement imager les vaisseaux sanguins capillaires d'un tissu vivant (2) susceptible de se déplacer, comprenant :
• une source de lumière (3) ;
• un dispositif optique (4) pour guider la lumière en direction du tissu vivant (2), la lumière étant absorbée par les érythrocytes des vaisseaux sanguins capillaires ;
• une caméra (5) pour acquérir au moins une séquence d'images qui met en évidence une région d'intérêt (6) des vaisseaux sanguins capillaires, sur une durée donnée, l'absorption de la lumière par les érythrocytes sur les images mettant en évidence les vaisseaux sanguins capillaires,
• un microscope (7) raccordé à la caméra (5),
• une unité de traitement et d'affichage (8), raccordée à la caméra (5),
• l'unité de traitement d'affichage étant configurée pour :
- sélectionner des images de la séquence, appelées « images sélectionnées », agencées dans l'ordre d'acquisition chronologique ;
- mélanger les images sélectionnées, pour décorréler temporellement les images sélectionnées, en les agençant dans un ordre mélangé différent de l'ordre chronologique ;
- réaligner spatialement les images sélectionnées agencées dans l'ordre mélangé ;
- générer une image projetée par projection des pixels des images sélectionnées réalignées, dans un empilement,
les valeurs projetées des pixels formant l'image projetée étant :
• des valeurs d'intensité minimale des pixels de toutes les images sélectionnées, la projection des valeurs d'intensité minimale des pixels représentant toutes les positions de tous les érythrocytes de toutes les images sélectionnées sur l'image projetée, ou
• des valeurs d'intensité moyenne des pixels de toutes les images sélectionnées, la projection des valeurs d'intensité moyenne représentant le flux moyen dans les vaisseaux capillaires sur l'image projetée, ou
• des valeurs d'intensité maximale des pixels de toutes les images sélectionnées, la projection des valeurs d'intensité maximale des pixels représentant les positions où le flux d'érythrocytes est stagnant.

2. Dispositif selon la revendication 1, dans lequel l'unité de traitement et d'affichage (8) est configurée pour réaligner spatialement les images sélectionnées dans l'ordre mélangé, sans prendre en considération un profil vasculaire commun fixe de toutes les images sélectionnées.

3. Dispositif selon les revendications 1 et 2, dans lequel l'unité de traitement et d'affichage (8) est configurée pour projeter au moins certaines des images sélectionnées dans leur ordre chronologique sur l'image projetée pour voir une vidéo du flux d'érythrocytes dans les vaisseaux sanguins capillaires.

4. Dispositif selon les revendications 1 à 3, dans lequel l'unité de traitement et d'affichage (8) est configurée pour mélanger les images sélectionnées de telle sorte que deux images successives dans l'ordre mélangé correspondent à des images qui sont séparées l'une de l'autre par au moins deux autres images successives dans l'ordre chronologique, de préférence par plus de 50 images dans l'ordre chronologique, de préférence correspondant à au moins une seconde en temps relevé.

5. Dispositif selon l'une quelconque des revendications 1 à 4, dans lequel l'unité de traitement et d'affichage (8) est configurée pour mélanger les images sélectionnées par :
- séparation de la séquence d'images sélectionnées en au moins deux sous-séquences,
- distribution des images sélectionnées des sous-séquences pour définir les images sélectionnées de l'ordre mélangé de telle sorte que les positions successives de l'ordre mélangé correspondent aux images sélectionnées des sous-séquences différentes.

6. Dispositif selon l'une quelconque des revendications 1 à 5, dans lequel le dispositif (1) comprend une unité de commande (9) couplée à la caméra (5), l'unité de commande (9) étant configurée pour commander à la caméra (8) d'acquérir une pluralité de séquences, chaque séquence étant acquise pendant une durée de temps donnée, et pour commander une longueur de temps entre chaque séquence.

7. Dispositif selon la revendication 6, dans lequel l'unité de traitement et d'affichage (8) est configurée pour calculer les images projetées des séquences respectives, agencées dans l'ordre chronologique d'acquisition des séquences, une image projetée par séquence, pour afficher une vidéo de l'évolution des vaisseaux sanguins capillaires avec les images projetées au moins une partie des images sélectionnées étant projetées dans leur ordre chronologique sur les images projetées pour voir une vidéo du flux d'érythrocytes dans les vaisseaux sanguins capillaires.

8. Dispositif selon l'une quelconque des revendications 6 à 7, dans lequel l'unité de commande (9) est configurée pour déplacer la caméra si la région d'intérêt se déplace sans retourner à sa position initiale, de manière à continuer d'observer la région d'intérêt après son déplacement par rapport à la position initiale dans le tissu vivant.

9. Dispositif selon les revendications 1 à 8, dans lequel, pour obtenir les images sélectionnées, l'unité de traitement et d'affichage (8) est configurée pour :
- calculer le niveau de gris de référence minimal de l'image de référence ;
- sélectionner les images de la séquence qui ont un niveau de gris minimal proche du niveau de gris de référence en définissant un critère tel qu'un nombre donné d'images, par exemple 50 % sont considérées comme proches de l'image de référence et conservées ;
- calculer l'intensité totale en ajoutant tous les pixels des premières images sélectionnées ;
- sélectionner les images parmi les premières images sélectionnées qui ont une intensité totale supérieure à un seuil, les secondes images sélectionnées qui sont sélectionnées étant les images sélectionnées.

10. Dispositif selon l'une quelconque des revendications 1 à 9, dans lequel l'unité de traitement et d'affichage (8) est configurée pour :
- mesurer l'intensité résultante de tous les pixels dans les vaisseaux sanguins capillaires sur l'image projetée par la projection de valeurs minimales, et
- calculer la profondeur des vaisseaux sanguins capillaires perpendiculaires aux plans des images ;
la profondeur étant fonction de l'intensité des pixels (qui est fonction du nombre d'érythrocytes dans la direction Z, qui se déplace dans les vaisseaux sanguins) de manière à déduire la section transversale des vaisseaux sanguins capillaires.

11. Dispositif selon l'une quelconque des revendications 1 à 10, dans lequel le réalignement spatial comprend le décalage spatial et/ou la rotation de chaque image sélectionnée par rapport à l'image sélectionnée préalable agencée dans l'ordre mélangé.

12. Dispositif selon l'une quelconque des revendications 1 à 11, dans lequel la caméra comprend un capteur d'image et un filtre optique qui permet la transmission au capteur d'image de lumière filtrée à des longueurs d'onde comprises entre 450 nm et 650 nm, de préférence entre 490 nm et 590 nm.

13. Dispositif selon l'une quelconque des revendications 1 à 12, dans lequel la séquence comprend au moins 30 images sélectionnées et de préférence au moins 100 images sélectionnées pour l'image projetée, la caméra étant configurée pour acquérir au moins 10 images de la séquence par seconde.

14. Méthode mise en œuvre par ordinateur pour automatiquement imager des vaisseaux sanguins capillaires d'un tissu vivant (2) susceptible de se déplacer, comprenant :
- l'éclairage du tissu vivant, la lumière étant partiellement absorbée par les érythrocytes des vaisseaux sanguins capillaires ;
- l'acquisition d'au moins une séquence d'images qui met en évidence une région d'intérêt (6) des vaisseaux sanguins capillaires, pendant une durée donnée,
- la sélection d'images de la séquence, appelées « images sélectionnées », agencées dans l'ordre d'acquisition chronologique ;
- le mélange des images sélectionnées, pour décorréler temporellement les images sélectionnées, en les agençant dans un ordre mélangé différent de l'ordre chronologique ;
- le réalignement spatial des images sélectionnées agencées dans l'ordre mélangé ;
- la génération d'une image projetée par projection des pixels des images sélectionnées réalignées, dans un empilement,
les valeurs projetées des pixels formant l'image projetée étant :
• des valeurs d'intensité minimale des pixels de toutes les images sélectionnées, la projection des valeurs d'intensité minimale des pixels représentant toutes les positions de tous les érythrocytes de toutes les images sélectionnées sur l'image projetée, ou
• des valeurs d'intensité moyenne des pixels de toutes les images sélectionnées, la projection des valeurs d'intensité moyennes représentant le flux moyen dans les vaisseaux capillaires sur l'image projetée, ou
• des valeurs d'intensité maximale des pixels de toutes les images sélectionnées, la projection des valeurs d'intensité maximale des pixels représentant les positions où le flux d'érythrocytes est stagnant.

15. Méthode selon la revendication 14, dans laquelle le réalignement spatial des images sélectionnées dans l'ordre mélangé est réalisé sans prendre en considération un profil vasculaire commun fixe de toutes les images sélectionnées.

16. Méthode selon les revendications 14 et 15, dans laquelle après le traitement de l'image projetée, la méthode comprend l'étape de projection d'au moins certaines des images sélectionnées dans leur ordre chronologique sur l'image projetée pour voir une vidéo du flux d'érythrocytes dans les vaisseaux sanguins capillaires.

17. Méthode selon l'une quelconque des revendications 15 et 16, dans laquelle le mélange comprend les deux sous-étapes suivantes :
- séparation de la séquence d'images sélectionnées en au moins deux sous-séquences,
- distribution des images sélectionnées des sous-séquences pour définir les images sélectionnées de l'ordre mélangé de telle sorte que les positions successives de l'ordre mélangé correspondent aux images sélectionnées de sous-séquences différentes.

18. Méthode selon les revendications 15 à 17, dans laquelle la caméra (8) est commandée pour acquérir une pluralité de séquences, chaque séquence étant acquise pendant une durée donnée, et pour attendre pendant une durée de temps donnée entre chaque séquence,
la méthode comprend une étape de calcul des images projetées des séquences respectives, agencées dans l'ordre chronologique d'acquisition des séquences, une image projetée par séquence, pour afficher une vidéo de l'évolution des vaisseaux sanguins capillaires avec les images projetées.

19. Méthode selon l'une quelconque des revendications 15 à 18, dans laquelle la méthode comprend les étapes suivantes :
- mesure de l'intensité des pixels dans un vaisseau sanguin capillaire sur l'image projetée, et
- calcul de la profondeur des vaisseaux sanguins capillaires perpendiculaires aux plans des images ;
la profondeur étant fonction de :
- l'intensité des pixels et
- la largeur des vaisseaux sanguins capillaires, pour déduire la section transversale du vaisseau sanguin capillaire.

20. Utilisation d'un dispositif selon l'une quelconque des revendications 1 à 13 pour l'imagerie *in vitro*/*in vivo* de vaisseaux sanguins capillaires d'un tissu vivant,
le tissu vivant étant choisi parmi :
- la membrane chorioallantoïque de poussin,
- une oreille de souris ou de rat,
- le mésentère de lapin,
- les vaisseaux de poisson-zèbre,
- le cerveau de poulet,
- la rétine de poulet,
- le système vasculaire limbique superficiel de poulet,
- le sac vitellin de poulet,
- et généralement tous les embryons se développant in ovo (reptiles, oiseaux) et ayant des organes extra embryonnaires similaires et des vaisseaux sanguins visibles (par exemple vaisseaux limbiques, vaisseaux intersomitiques etc.),
- la rétine.
